# FASCICULE DE BREVET EUROPEEN

(11) **EP 0 481 147 B1**
(45) Date de publication et mention de la délivrance du brevet: **10.06.1998**
(21) Numéro de dépôt: 90402894.1
(22) Date de dépôt: 16.10.1990
(51) Int. Cl.: C12P 7/04, C12P 7/24

(54) **Procédé de synthèse du cis-3-hexene-1-ol à partir d'acide gras insaturé**
Verfahren zur Herstellung von Cis-3-hexen-1-ol aus ungesättigten Fettsäuren
Process for the preparation of cis-3-hexene-1-ol from unsaturated fatty acids

(43) Date de publication de la demande: 22.04.1992
(73) Titulaire: PERNOD-RICARD, F-75008 Paris (FR)
(72) Inventeur: Brunerie, Pascal, F-94100 Saint Maur (FR)
(74) Mandataire: Warcoin, Jacques

(56) Documents cités:
- CHEMICAL ABSTRACTS, vol. 107, no. 3, 20 juillet 1987, page 375, Résumé no. 20795u, Columbus, Ohio, US; R.G. BERGER et al.: "C6-aldehyde formation from linolenic acid in fruit cells cultured in vitro", && PLANT CELL TISSUE ORGAN CULT. 1987, 8(2), 147-51
- CHEMICAL ABSTRACTS, vol. 109, no. 9, 29 août 1988, page 380, résumé no. 70056r, Columbus, Ohio, US; G. BOCK et al.: "The reduction of cinnamaldehyde and insaturated acids by Botrytis cinerea", & Z. LEBENSM.-UNTERS. FORSCH. 1988, 186(1), 33-5
- Connaissance Vigne Vin, 1981, 15, No. 4, p. 269-286.
- Le Vin de l'analyse à l'élaboration, troisième édition, Tec-Doc - Lavoisier, Paris, p. 136-141
- Plant and Cell Physiol., 18, 1977, p. 107-116
- Lebensm.-Wiss. u. Technol., 19, 1986, p. 152-155

## Description

La présente invention a pour objet un procédé de synthèse du cis-3-hexene-1-ol à partir d'acide gras insaturé. Elle a plus particulièrement pour objet un procédé biologique d'obtention d'alcool spécifié ci-dessus.

De nombreux aldéhydes et alcools en C6 sont produits lors du broyage des tissus végétaux et possèdent des propriétés organoleptiques et physiologiques très intéressantes.

Parmi ces composés, le cis-3-hexenol est largement employé dans les compositions aromatiques pour leur donner une note "verte et fraîche".

L'obtention de cet alcool est difficile, que ce soit par les voies biologiques ou par les voies de la synthèse organique.

De nombreuses études ont été menées pour déterminer et mesurer la faculté de certains tissus végétaux à former du cis-3-hexenol.

Parmi ces études, il convient de citer l'étude du Professeur Peter Schreier qui a fait l'objet de l'article (1986) "C6 volatils in homogenates from Green Leaves : localization of hydroperoxidase lyase activity", Lebensm. Wiss.u.Technol. 19, 152-156. Cette étude a montré que de nombreux tissus végétaux, notamment les feuilles, étaient susceptibles de produire des quantités mesurables de cis-3-hexenol. Plus particulièrement, il a été montré que les fanes de radis et de vignes pouvaient produire jusqu'à 80 mg de cis-3-hexenol par kg de matériel végétal humide.

L'article mentionné ci-dessus expose la voie enzymatique la plus généralement admise pour passer d'acides gras insaturés, notamment de l'acide linolénique, au cis-3-hexenal puis au cis-3-hexenol. Ainsi, une lipoxygénase catalyserait la formation d'un péroxyde qui serait ensuite ouvert par une hydropéroxyde lyase pour fournir les aldéhydes volatils en C6. Une aldéhyde réductase permettrait ensuite la réduction des aldéhydes en alcool correspondant.

Les rendements évoqués ci-dessus, bien qu'ils démontrent l'existence d'un système enzymatique permettant de fabriquer le cis-3-hexenol à partir d'acide gras insaturé, sont insuffisants pour permettre une exploitation rentable de ces systèmes enzymatiques.

C'est la raison pour laquelle, il a été proposé d'ajouter au broyat de feuilles de l'acide linolénique, précurseur naturel des aldéhydes et alcools en C6, pour augmenter la production du cis-3-hexenol et cis-3-hexenal.

Les brevets US 4 769 243 et 4 806 879 donnent une bonne illustration d'une telle technique. L'article de Connaissance Vigne Vin 1981, 15 n° 4 pp 269-286 décrit la production de cis-3-hexenal et trans-2-hexenal dans un broyat de raisin et fait état de leur réduction subséquente en cis-3-hexenol et trans-2-hexenol, soit par une alcool deshydrogénase constitutive du raisin, soit par l'action de la levure au cours de la fermentation alcoolique transformant le jus de raisin en vin. Ce document montre également que c'est toujours la forme trans-2 qui est prédominante (trans-2-hexenal et/ou trams-2-hexenol).

Toutefois, ces techniques ne permettent qu'un rendement en alcool désiré médiocre.

C'est pourquoi, un des buts de la présente invention est de fournir un procédé de synthèse du cis-3-hexenol qui permette une amélioration significative par rapport aux résultats obtenus dans les procédés exposés ci-dessus.

Ce but, et d'autres qui apparaîtront par la suite, sont atteints au moyen d'un procédé de synthèse du cis-3-hexene-1-ol dans lequel ladite synthèse est réalisée à partir d'acide gras insaturé par l'action combinée d'un système naturel d'enzyme(s) permettant l'oxydation dudit acide gras en cis-3-hexenal et d'une levure permettant la réduction du cis-3-hexenal en cis-3-hexenol comme indiqué dans le schéma I à l'exemple 1.

Ainsi, au cours de l'étude qui a mené à la présente invention, il a pu être montré que l'addition de l'acide linolénique conduisait à une légère augmentation de la teneur en aldéhyde cis-3-hexenal précurseur de l'alcool désiré et à une très forte augmentation de la teneur en trans-2-hexenal, lequel est un sous-produit d'isomérisation moins intéressant.

Après une étude plus poussée, il est apparu que la concentration en cis-3-hexenal passe par un maximum, et, progressivement, décroît plus au profit de son isomère, le trans-2-hexenal qu'au profit de son composé réduit, le cis-3-hexenol.

De plus, selon la présente invention, il a été démontré que l'addition de levure permettait de privilégier la transformation du cis-3-hexenal en cis-3-hexenol, ce dernier composé étant plus stable que son homologue aldéhydique, aucune isomérisation en trans-2-hexenol n'est alors observée.

Ces levures peuvent être toute levure présentant les caractéristiques suivantes : activité alcool déshydrogénase importante permettant de réduire les composés carbonylés en alcools correspondants.

On utilise avantageusement une levure du type saccharomyces, de préférence Saccharomyces cerevisiae.

Les résultats obtenus au cours de l'étude qui a mené à la présente invention ont tous été réalisés en utilisant des levures vendues dans le commerce telles que notamment celles vendues sous la marque LEVULINE par les LABORATOIRES OENOLOGIQUES DE FRANCE.

Avantageusement, la réaction est réalisée dans un milieu de culture favorable aux systèmes enzymatiques et aux levures. Les milieux de culture favorables aux levures sont bien connus de l'homme de métier et n'ont pas besoin d'être détaillés dans la présente decription.

Il convient toutefois de noter, que lorsqu'on utilise des broyats de feuilles comme système naturel d'enzyme, la pulpe obtenue par mélange en milieux aqueux dudit broyat constitue un milieu de culture convenable.

Pour mettre en oeuvre le procédé selon la présente invention, il est souhaitable d'effectuer la séquence d'étapes suivantes :
a) introduction dudit acide gras insaturé et dudit système enzymatique dans un milieu de culture aqueux ;
b) introduction de la levure dans ledit milieux de culture.

Pour minimiser la production de composés indésirés, tels que le trans-2-hexenal et le trans-2-hexenol, il est souhaitable que l'introduction de ladite levure soit effectuée au plus tard au moment où la concentration en cis-3-hexenal dans ledit milieu est maximale.

Comme la courbe de la teneur en cis-3-hexenal en fonction du temps est une courbe relativement aplatie, la période de temps pendant laquelle il n'y a pas grand dommage à attendre pour ajouter la levure est assez étendue. Pour obtenir un meilleur résultat, il est préférable qu'au moment de l'introduction de la levure dans ledit milieu de culture, ladite levure soit en phase de croissance. Avantageusement, le pH du milieu de culture est maintenu entre les valeurs arrondies 2 et 7, de préférence entre 3 et 6. La température à laquelle doit avoir lieu la synthèse est limitée par les cinétiques des systèmes enzymatiques et des levures. En outre, à partir d'une certaine élévation de température, les levures sont tuées et ne sont plus utilisables. C'est pourquoi, en général, on utilise une température comprise entre 0 et 60°C avantageusement, entre 15 et 40°C, en général aux alentours de la température ambiante, c'est-à-dire entre 17 et 30°C.

Toutefois, il peut être intéressant de mettre en oeuvre le système enzymatique à des températures comprises entre 0°C et 20°C, pour obtenir une excellente sélectivité de la production en dérivés du cis-3-hexene par rapport à ceux du trans-2-hexene.

Le système enzymatique permettant d'obtenir une quantité importante de volatiles en C6 peut provenir d'un grand nombre de tissus végétaux. Ainsi, il peut provenir des feuilles de vigne, du tabac, de chicorée (notamment des espèces produisant des endives), de poireaux, de navet, de choux-rave et de divers radis. De tels systèmes existent également dans les tissus du soja et de différentes variétés de Rumex.

On peut également citer les tissus de plantes fournissant des baies telles que les feuilles de fraisiers.

Toutefois, les systèmes enzymatiques préférés sont ceux que l'on peut obtenir à partir des fanes de radis, de vignes et de Rumex.

Ces systèmes enzymatiques sont en partie étroitement associés à la membrane des chloroplastes.

Selon la présente invention, le procédé de synthèse le plus utilisé est celui dans lequel le système enzymatique est introduit sous forme de fanes de radis broyées.

En général, ces systèmes enzymatiques utilisent les acides gras constitutifs qui sont susceptibles d'être transformés en aldéhyde en C6. Il est cependant préférable d'ajouter certaines quantités d'acides gras comme précurseur pour augmenter le rendement. Ces acides gras sont en général des acides linoléniques ou linoléiques. Cela peut toutefois être tout acide gras insaturé à chaîne linéaire, possédant au moins trois insaturations, lesquelles insaturations sont situées en position 6, 9 et 12 à partir de l'extrémité CH3 de la chaîne carbonée.

Lorsque l'on utilise comme système enzymatique une masse végétale obtenue par broyage de tissus adéquats, il est avantageux que l'acide gras introduit ait une concentration comprise entre 0,1 et 2 % en poids de la masse végétale humide. Celle-ci est en solution à 10 % dans de l'eau distillée.

Les acides gras peuvent être introduits sous forme acide ou sous la forme d'un sel dont le cation associé à l'acide gras est acceptable pour les levures et pour le système enzymatique. Il est également souhaitable que les sels d'acide gras soient solubles en milieux aqueux. Les sels les plus couramment utilisés sont les sels des acides gras avec les métaux alcalins et avec le cation ammonium. L'acide linolénique peut être introduit soit sous forme pure, soit en mélange avec d'autres acides gras. En particulier, il est possible d'utiliser des mélanges d'acide gras provenant de la saponification d'huiles riches en acide linolénique, telle que l'huile de lin.

La saponification peut être réalisée soit par voie chimique, soit par voie biologique, par exemple au moyen de lipases.

Les exemples non limitatifs suivants illustrent l'invention.

### Protocole opératoire général des exemples

20 g de feuilles de radis (de Raphanus Sativus L., variés 18 jours) sont broyées puis mises en suspension dans 0,200 litre d'eau. On ajoute simultanément la quantité d'acide linolénique testé. Sauf lorsque cela est indiqué autrement, la température est la température ambiante (20°C environ), les levures (1 g de levures régénérées) sont ajoutées après 45 mn d'incubation ; sauf quand cela est indiqué autrement, le pH est le pH naturel.

Dans les tableaux ci-après, on a exprimé les résultats en quantité de volatiles formés (en mg) par rapport au poids de matériel végétal frais mis en oeuvre au cours du procédé, ceci pour des raisons de commodité de reproductibilité et de compréhension. Ces résultats peuvent être exprimés par rapport à la matière sèche mise en oeuvre, sachant que les mesures qui ont été effectuées montrent que les feuilles de radis fraîches contiennent 8,5 ± 0,5 % de matières sèches.

Les mesures ont été réalisées en chromatographie en phase gazeuse, selon la méthode décrite ci-après.

### Extraction et dosage des volatiles

Après incubation, on ajoute au milieu 200 ml d'eau et le standard interne.

Le milieu est ensuite soumis à un entraînement à la vapeur. 250 ml de distillat sont récupérés et extraits avec trois fois 100 ml de penthane ether (2:1).

Après séchage et concentration à 1 ml, l'extrait est analysé en chromatographie en phase gazeuse comme décrit ci-dessous :
. Appareil :
   Chomatographie HEWLETT PACKARD 5890 équipé d'un injecteur split/splitless et d'un détecteur à ionisation de flamme.
. Colonne :
   FFAP HEWLETT PACKARD 50 m - diamètre : 0,32 mm - Epaisseur du film : 0,52 µm,
. Conditions d'analyse :
   Débit colonne He : 1 2 ml/mn, make up : 30 ml/mn, air : 350 ml/mn, hydrogène : 30 ml/mn.
Température de l'injecteur et du détecteur : 250°C.
Programme 50°C isotherme 3 minutes, 3°C/mn jusqu'à 120°C, puis 2°C/mn jusqu'à 220°C, et isotherme à 220°C pendant 60 minutes.

### Exemple 1 : essais de référence

En mettant en oeuvre le protocole opératoire ci-dessus, différents essais ont été menés pour déterminer les différents paramètres ayant une influence sur la production du cis-3-hexenal et du cis-3-hexenol par les tissus de radis.

Les principaux paramètres examinés ont été la température et le pH. Pour ce dernier paramètre, le pH naturel du milieu après broyage du tissu végétal permet d'obtenir les meilleurs résultats.

Les différentes données rassemblées dans le tableau I ci-après montrent d'une part, que les aldéhydes et alcools en C6 se forment très rapidement et d'autre part, qu'un abaissement de température significatif, bien que réduisant d'une manière importante la cinétique, permet de privilégier la formation du cis-3-hexenol par rapport au trans-2-hexenal et au trans-2-hexenol en évitant l'évolution du cis-3-hexenal vers le produit thermodynamiquement le plus stable: le trans-2-hexenal.

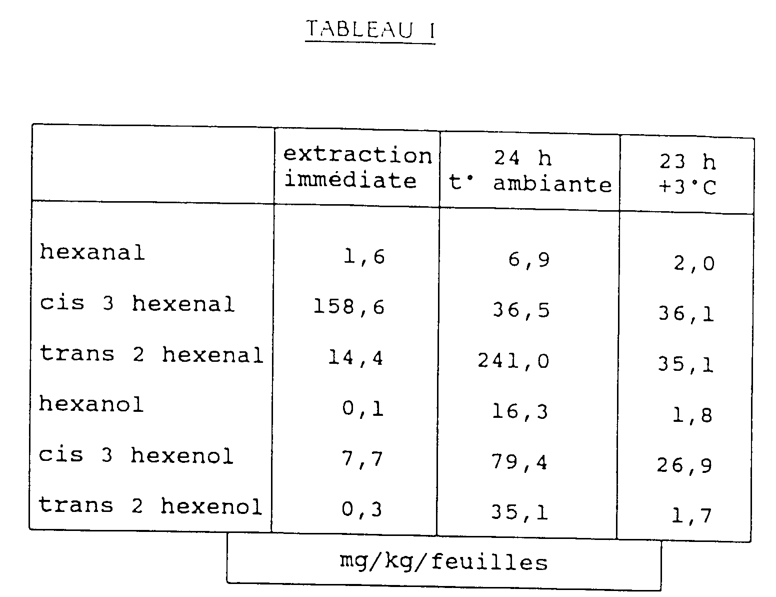

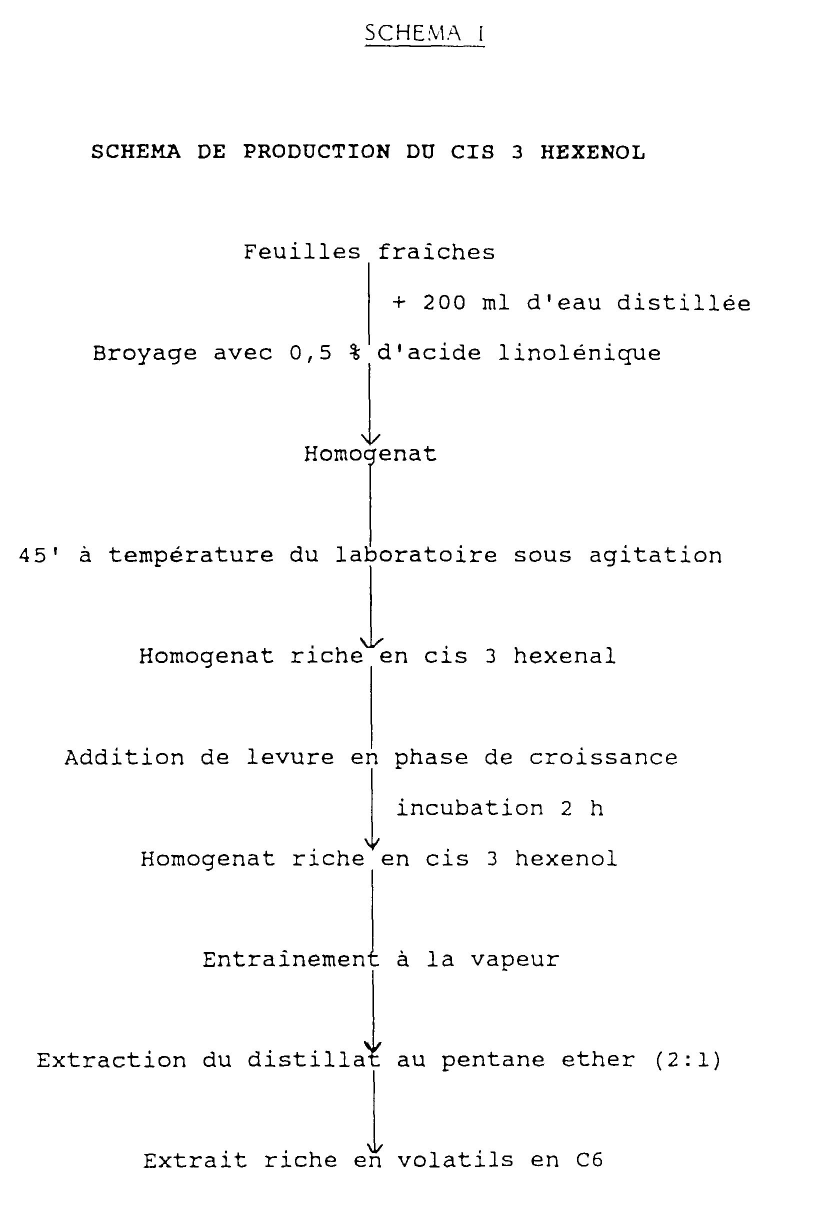

### Exemple II - Addition d'acide linolénique

Il apparaît du tableau II ci-après, que l'addition d'acide linolénique permet d'augmenter considérablement la teneur en aldéhydes produite tandis que les teneurs en cis-3-hexenal diminuent. De l'examen de ce tableau, on déduit que l'addition de levures sèches suivie de 2 heures d'incubation, permet d'augmenter très significativement la teneur en cis-3-hexenol par réduction enzymatique du cis-3-hexenal. Ainsi, le cis-3-hexenal, aldéhyde instable, formé grâce à l'activité lipoxygénase libérée par le broyage du tissu végétal, est transformé en cis-3-hexenol, composé stable, évitant ainsi la formation d'isomères tels que le trans-2-hexenol.

**TABLEAU II**

| BROYAGE DES FEUILLES EN PRESENCE DE PRECURSEUR : ACIDE LINOLENIQUE | | | |
|---|---|---|---|
| Quantité d'acide linolénique - Temps à la temperature ambiante | 0,5 % 23 h | 0,6 % 23 h | 0,5 % 2 h * |
| hexanal | 1,7 | 2,7 | 1,1 |
| cis 3 hexenal | 57,7 | 76,0 | 15,4 |
| trans 2 hexenal | 319,4 | 359,3 | 99,2 |
| hexanol | 1,0 | 0,2 | 10,9 |
| cis 3 hexenol | 7,5 | 4,8 | 379,6 |
| trans 2 hexenol | 1,6 | 0,3 | 37,4 |
| | mg/kg/feuilles | | |

| | | | |
|---|---|---|---|
| * +2 h levures sèches | | | |

Le tableau III rassemble les résultats obtenus avec une quantité variable d'acide linolénique. Il apparaît ainsi que la quantité optimale d'acide linolénique se situe aux alentours de 0,5, 1 %. Au delà de ces valeurs, l'acide linolénique devient très probablement inhibiteur de l'activité lypoxygénase.

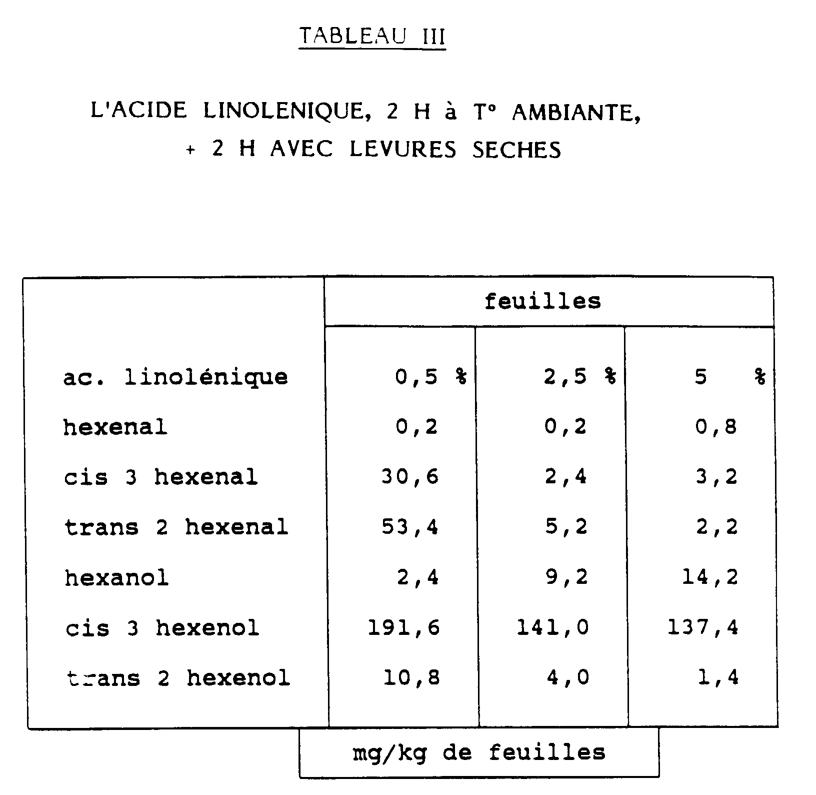

### Exemple 3 - Rôle des levures

Le tableau IV suivant rassemble les résultats obtenus à partir d'essais dans lesquels on a fait varier la teneur en acide linolénique, ainsi que la qualité des levures introduites. Les expériences ont été réalisées après une incubation d'une heure, suivie d'une adjonction de levure que l'on laisse agir pendant 2 heures. Les levures ont été introduites sous forme de levure sèche ou sous forme de levure en phase de croissance.

Ce tableau IV montre que les levures préalablement régénérées, c'est-à-dire en phase de croissance, développent une activité alcool déshydrogénase importante permettant d'obtenir jusqu'à 550 mg/kg de cis-3-hexenol, ce qui représente, comme l'indique le tableau V, 95,5 % des volatiles totaux de l'extrait. Le même procédé a été testé en utilisant Rumex acetosa comme matériel végétal, ce qui a permis d'obtenir des résultats comparables (tableau V).

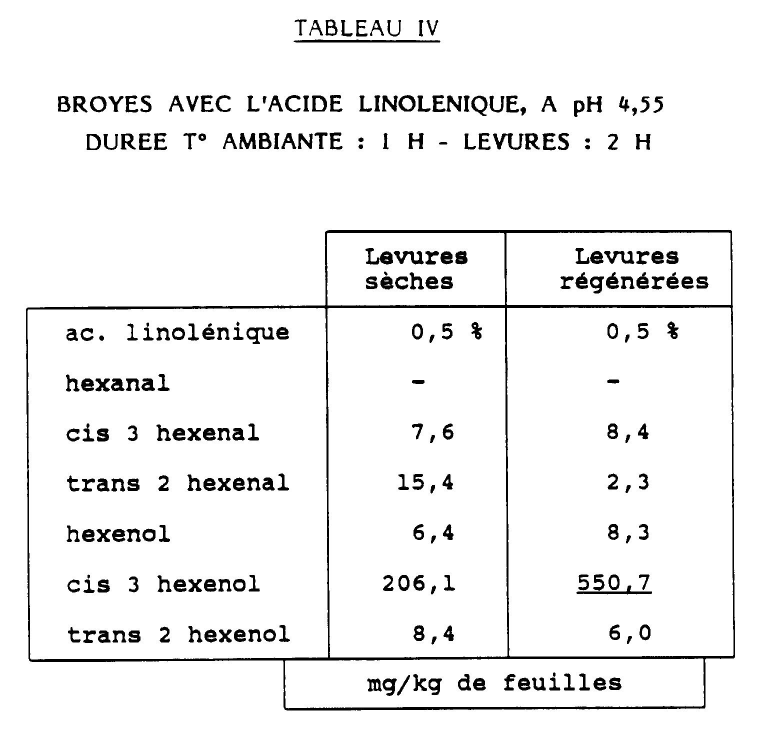

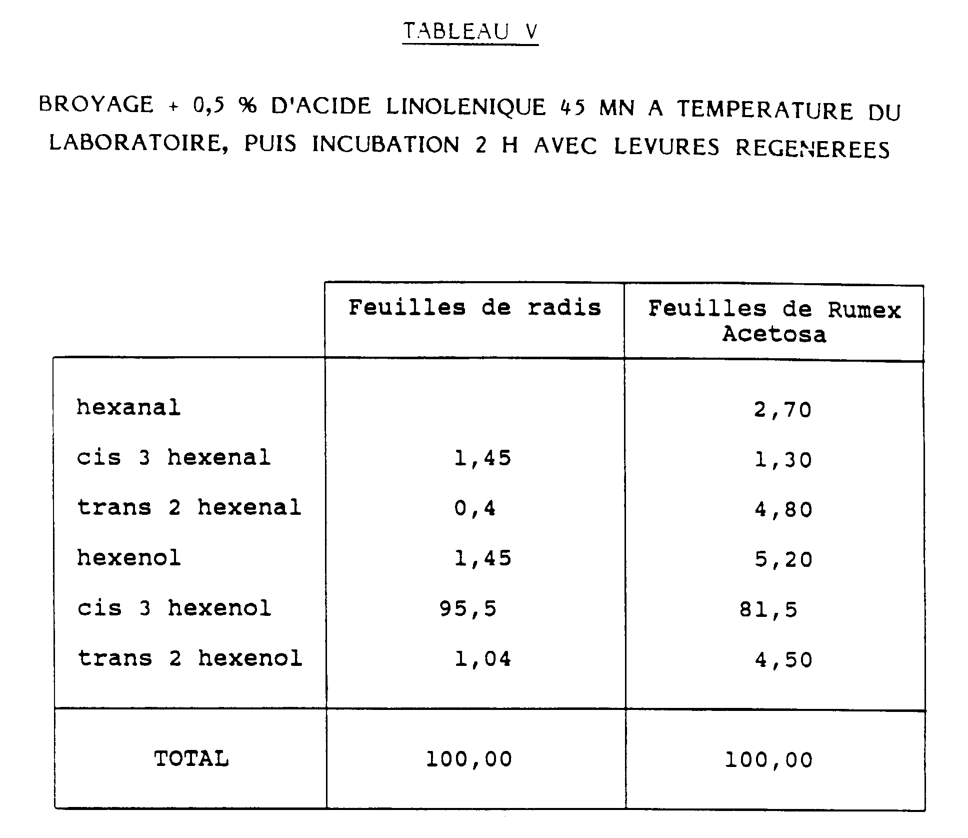

### Exemple 4 - Etude cinétique

Le tableau VI ci-après rassemble les résultats obtenus à partir de 0,5 % d'acide linolénique et un broyat de feuilles de radis à température ambiante. Ces résultats montrent que la somme des teneurs en cis-3-hexenal et en cis-3-hexenol est maximum dans les conditions de la réaction aux environs de 45 mn. Au delà de ce temps d'incubation, le cis-3-hexenal, instable, s'isomérise au profit du trans-2-hexenal, les quantités de cis-3-hexenol restant stables.

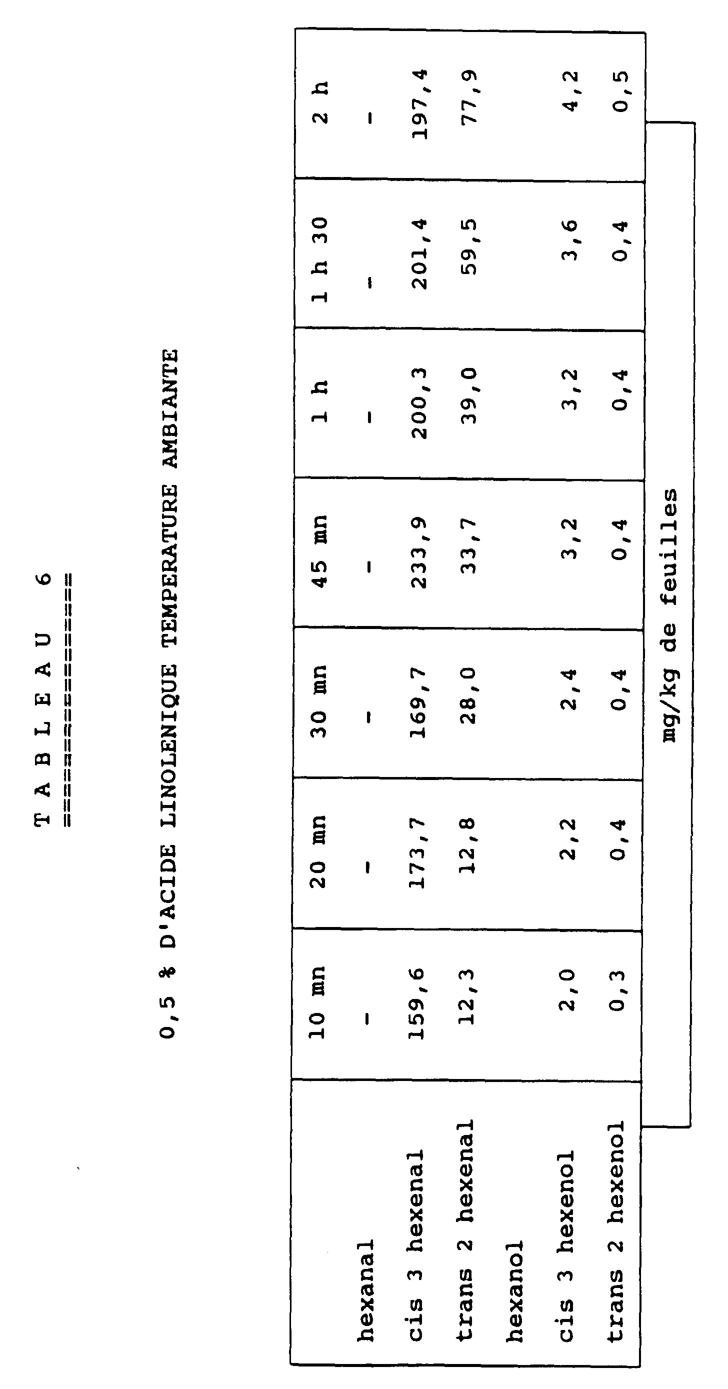

## Revendications

1. Procédé de synthèse du cis-3-hexene-1-ol à partir d'acide gras insaturé, caractérisé par le fait que ladite synthèse est realisée dans un milieu de culture à partir de ce dernier par l'action combinée d'un système naturel d'enzyme(s) permettant l'oxydation dudit acide gras en cis-3-hexenal et d'une levure ajoutée dans ledit milieu de culture permettant la réduction du cis-3-hexenal en cis-3-hexenol.

2. Procédé de synthèse selon la revendication 1, caractérisé par le fait qu'il comporte les étapes suivantes :
a) introduction dudit acide gras insaturé et dudit système enzymatique dans un milieu de culture aqueux ;
b) introduction de la levure dans ledit milieux de culture.

3. Procédé de synthèse selon la revendication 2, caractérisé par le fait que l'introduction b) de ladite levure est effectuée au plus tard au moment où la concentration en cis-3-hexenal dans ledit milieu est maximale.

4. Procédé de synthèse selon l'une des revendications 2 et 3, caractérisé par le fait qu'au moment de l'introduction, ladite levure est en phase de croissance.

5. Procédé selon l'une des revendications 2 à 4, caractérisé par le fait que le pH dudit milieu de culture est maintenu entre 2 et 7, de préférence entre 3 et 6.

6. Procédé selon l'une des revendications 2 à 5, caractérisé par le fait que la température du milieu de culture est maintenue entre 15 et 40°C

7. Procédé selon l'une des revendications 2 à 6, caractérisé par le fait que ledit système enzymatique est introduit sous la forme de fanes de radis ou de rumex.

8. Procédé selon l'une des revendications 1 à 7, caractérisé par le fait que ledit acide gras est l'acide linolénique.

9. Procédé selon la revendication 8, caractérisé par le fait que ledit acide gras est l'acide linolénique et qu'il est introduit à une concentration comprise entre 0,1 et 2 % en poids de la masse végétale.

10. Procédé selon l'une des revendications 1 à 9, caractérisé par le fait qu'il comporte en outre une étape de récupération du cis-3-hexene-1-ol par entraînement à la vapeur.

## Patentansprüche

1. Verfahren zum Synthetisieren von cis-3-Hexen-1-ol aus einer ungesättigten Fettsäure, dadurch gekennzeichnet, daß die genannte Synthese in einem Kulturmedium durchgeführt wird, indem man von letzterer ausgeht und sie der kombinierten Einwirkung eines natürlichen Enzym-Systems, das die Oxidation der genannten Fettsäure zum cis-3-Hexenal erlaubt, und einer zu dem Kulturmedium gefügten Hefe, welche die Reduktion des cis-3-Hexenals zum cis-3-Hexenol erlaubt, aussetzt.

2. Syntheseverfahren nach Anspruch 1, dadurch gekennzeichnet, daß es die folgenden Stufen umfaßt:
a) Einführung der genannten ungesättigten Fettsäure und des genannten Enzymsystems in ein wäßriges Kulturmedium; und
b) Einführung von Hefe in das genannte Kulturmedium.

3. Syntheseverfahren nach Anspruch 2, dadurch gekennzeichnet, daß die Einführung (b) der genannten Hefe spätestens in dem Augenblick durchgeführt wird, in dem die Konzentration an cis-3-Hexenal in dem genannten Medium maximal ist.

4. Syntheseverfahren nach einem der Ansprüche 2 und 3, dadurch gekennzeichnet, daß die genannte Hefe im Augenblick ihrer Einführung sich in ihrer Wachstumsphase befindet.

5. Verfahren nach einem der Ansprüche 2 bis 4, dadurch gekennzeichnet, daß der pH-Wert des genannten Kulturmediums zwischen 2 und 7, vorzugsweise zwischen 3 und 6, gehalten wird.

6. Verfahren nach einem der Ansprüche 2 bis 5, dadurch gekennzeichnet, daß die Temperatur des Kulturmediums zwischen 15 und 40°C gehalten wird.

7. Verfahren nach einem der Ansprüche 2 bis 6, dadurch gekennzeichnet, daß das genannte Enzymsystem in Form von Rettichkraut oder Ampferkraut (Rumex-Kraut) eingeführt wird.

8. Verfahren nach einem der Ansprüche 1 bis 7, dadurch gekennzeichnet, daß die genannte Fettsäure Linolensäure ist.

9. Verfahren nach Anspruch 8, dadurch gekennzeichnet, daß die genannte Fettsäure die Linolensäure ist und daß sie in einer Konzentration zwischen 0,1 und 2 Gew.-%, bezogen auf das Gewicht der Pflanzenmasse, eingeführt wird.

10. Verfahren nach einem der Ansprüche 1 bis 9, dadurch gekennzeichnet, daß es außerdem eine Stufe der Gewinnung von cis-3-Hexen-1-ol durch Mitreißen im Dampf umfaßt.

## Claims

1. Process for the synthesis of cis-3-hexen-1-ol from unsaturated fatty acid, characterized in that the said synthesis is performed in a culture medium starting with the latter fatty acid by the combined action of a natural system of enzyme(s) which allows the said fatty acid to be oxidized into cis-3-hexenal and of a yeast added to the said culture medium which allows the cis-3-hexenal to be reduced into cis-3-hexenol.

2. Synthetic process according to Claim 1, characterized in that it contains the following steps:
a) introduction of the said unsaturated fatty acid and of the said enzymatic system into an aqueous culture medium;
b) introduction of the yeast into the said culture medium.

3. Synthetic process according to Claim 2, characterized in that the introduction b) of the said yeast is carried out, at the very latest, at the moment when the concentration of cis-3-hexenal in the said medium is at a maximum.

4. Synthetic process according to either of Claims 2 and 3, characterized in that at the moment of the introduction, the said yeast is in growth phase.

5. Process according to one of Claims 2 to 4, characterized in that the pH of the said culture medium is maintained between 2 and 7, preferably between 3 and 6.

6. Process according to one of Claims 2 to 5, characterized in that the temperature of the culture medium is maintained between 15 and 40°C.

7. Process according to one of Claims 2 to 6, characterized in that the said enzymatic system is introduced in the form of radish tops or dock tops.

8. Process according to one of Claims 1 to 7, characterized in that the said fatty acid is linolenic acid.

9. Process according to Claim 8, characterized in that the said fatty acid is linolenic acid and that it is introduced at a concentration between 0.1 and 2 % of the weight of the plant bulk.

10. Process according to one of Claims 1 to 9, characterized in that it additionally contains a step of recovery of the cis-3-hexen-1-ol by vapour entrainment.
